# EUROPEAN PATENT SPECIFICATION

(11) **EP 3 089 576 B1**
(45) Date of publication and mention of the grant of the patent: **17.04.2019**
(21) Application number: 14824205.0
(22) Date of filing: 05.12.2014
(51) Int. Cl.: A01G 22/00

(54) **METHODS AND COMPOSITIONS FOR PRODUCTION OF WATERMELON FRUIT**
VERFAHREN UND ZUSAMMENSETZUNGEN ZUR HERSTELLUNG VON WASSERMELONEN
PROCÉDÉS ET COMPOSITIONS POUR LA PRODUCTION DE FRUIT DE PASTÈQUE

(30) Priority: 31.12.2013 US 201314145509
(43) Date of publication of application: 09.11.2016
(73) Proprietor: Syngenta Participations AG, 4058 Basel (CH)
(72) Inventor: LIERE, Dean Geoffery, Gilroy, CA 95020-8033 (US); BRUSCA, James P., Woodland, CA 95695 (US); BOGARD, Jeannine R., Gilroy, CA 95020 (US)
(74) Representative: Syngenta International AG
(86) International application number: PCT/US2014/068789
(87) International publication number: WO 2015/102810

(56) References cited:
- US-A1- 2011 203 501
- US-A1- 2013 152 223
- US-B1- 8 212 118
- FREEMAN J H ET AL: "Performance of Selected Diploid Watermelon Pollenizers", INTERNET CITATION, October 2013 (2013-10), pages 1-4, XP008166211, Retrieved from the Internet: URL:http://edis.ifas.ufl.edu/hs332 [retrieved on 2013-12-09]

## Description

### FIELD OF THE INVENTION

The present invention relates to compositions and methods for providing watermelon fruit, optionally over an extended season.

### BACKGROUND OF THE INVENTION

Seedless watermelon plants are produced by crossing a tetraploid watermelon variety with a diploid watermelon variety. Such a cross results in triploid hybrid plants that are sterile due to the uneven pairing of the chromosomes. Therefore, to produce fruit, a seedless watermelon variety must be fertilized by the pollen of a seeded diploid watermelon variety. While seedless watermelons are quite popular with consumers, their production in the home garden can be limited by the need to grow a separate pollenizer variety along side of the seedless variety. Further, seedless watermelon varieties generally require special conditions for germination, emergence and early plant development making them difficult to start from seed in a typical garden. Consequently, often being unaware of the particular cultural requirements of seedless watermelon varieties, the home gardener can become frustrated in their efforts to produce seedless watermelon in a home garden setting.

Thus, the present invention overcomes the shortcomings in the field by providing compositions and methods that assist the grower (*e.g.,* home gardener; chef, and the like) in producing a crop of seedless watermelon fruits, optionally with the added benefit of production of said fruits over an extended season. A method of planting triploid seedless watermelon seeds is known from US2011/0203501 A1.

### SUMMARY OF THE INVENTION

According to the invention there is provided method for producing watermelon fruit as in the appended independent claim 1.

The skilled person will undoubtedly recognize that all aspects of the invention disclosed above and below can also be put into practice when using vegetatively-propagated watermelon materials instead of seedlings. In particular, vegetative cuttings or plants derived from tissue or cell culture can alternatively be used without affecting the essence of the invention.

The foregoing and other aspects of the present invention will now be described in more detail with respect to other embodiments described herein. It should be appreciated that the invention can be embodied in different forms and should not be construed as limited to the embodiments set forth herein. Rather, these embodiments are provided so that this disclosure will be thorough and complete, and will fully convey the scope of the invention to those skilled in the art.

### BRIEF DESCRIPTION OF THE DRAWINGS

**Fig. 1** shows an exemplary plug tray.
**Fig. 2** shows a closer view of the plug tray of **Fig. 1****.**
**Fig. 3** shows an exemplary arrangement of seedlings in a container of the invention in which two seedlings of the same seedless watermelon variety are planted with two seedlings of the same seeded pollenizer watermelon variety.
**Fig. 4** shows an exemplary arrangement of seedlings in a container of the invention in which two seedlings of different seedless watermelon varieties are planted with two seedlings of the same seeded pollenizer watermelon variety.
**Fig. 5A-5F** each show exemplary containers of the invention comprising at least two seedlings of a seeded pollenizer watermelon variety and at least two seedlings of a seedless watermelon variety. In this photograph, the seeded variety is exemplified by 'Quetzali' (early season) and the seedless varieties are exemplified by 'Sweet Gem' (mid-season) and 'Dorin' ('RWT8212') (early season).
**Fig. 6A-6C** each show an exemplary group of plants from a single container of the invention after planting and establishment in the ground. As exemplified here, the three different watermelon varieties have three different times to maturity, flowering and fruit production and therefore as a group produce fruit throughout the season (exemplified in this photograph with 'Quetzali' as the seeded pollenizer and 'Sweet Gem' and 'Dorin' as the seedless varieties).
**Fig. 7** shows exemplary watermelon fruits, seedless and seeded, that can be produced over an extended season using the containers and methods of this invention. In this photograph, the seeded pollenizer variety is exemplified by 'Quetzali' (early season) and the seedless varieties are exemplified by 'Sweet Gem' (mid-season) and 'Dorin' (early season).

### DETAILED DESCRIPTION OF THE INVENTION

Unless the context indicates otherwise, it is specifically intended that the various features of the invention described herein can be used in any combination.

Moreover, the present invention also contemplates that in some embodiments of the invention, any feature or combination of features set forth herein can be excluded or omitted. To illustrate, if the specification states that a composition comprises components A, B and C, it is specifically intended that any of A, B or C, or a combination thereof, can be omitted and disclaimed singularly or in any combination.

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

As used in the description of the invention and the appended claims, the singular forms "a," "an" and "the" are intended to include the plural forms as well, unless the context clearly indicates otherwise.

As used herein, "and/or" refers to and encompasses any and all possible combinations of one or more of the associated listed items, as well as the lack of combinations when interpreted in the alternative ("or").

The term "about," as used herein when referring to a measurable value such as a dosage, an amount or a time period and the like, means variations of ± 20%, ± 10%, ± 5%, ± 1%, ± 0.5%, or even ± 0.1% of the specified amount (*e.g*., the time between the opening of the first flowers of an early season variety as compared to the opening of the first flowers of a mid-season variety).

As used herein, phrases such as "between X and Y" and "between about X and Y" should be interpreted to include X and Y. As used herein, phrases such as "between about X and Y" mean "between about X and about Y." As used herein, phrases such as "from about X to Y" mean "from about X to about Y".

The term "comprise," "comprises" and "comprising" as used herein, specify the presence of the stated features, integers, steps, operations, elements, and/or components, but do not preclude the presence or addition of one or more other features, integers, steps, operations, elements, components, and/or groups thereof.

As used herein, the transitional phrase "consisting essentially of" means that the scope of a claim is to be interpreted to encompass the specified materials or steps recited in the claim and those that do not materially affect the basic and novel characteristic(s) of the claimed invention. Thus, the term "consisting essentially of" when used in a claim of this invention is not intended to be interpreted to be equivalent to "comprising".

As used herein, the phrase "produces male flowers in sufficient time to pollenize female flowers" means that the plants of a seeded pollenizer watermelon variety reach a stage of development in which the first male flowers produced open prior to or about the same time (on average) as the opening of the first female flowers produced by a seedless watermelon variety(ies), such that the male flowers of the seeded pollenizer watermelon variety are able to pollinate the female flowers of the seedless variety(ies). Thus, in particular embodiments, the first male flowers produced by a seeded pollenizer watermelon variety open at least about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about one day, about two days, about three days, about four days, about five days, about six days, about seven days, about eight days, about nine days, about ten days, about eleven days, or about twelve days, or any range therein, prior to the opening of the first female flowers produced by a seedless watermelon variety(ies). Thus, in representative embodiments, in at least one seeded pollenizer watermelon variety, flower production (including flower opening) is initiated prior to flower production (including flower opening) in at least one seedless watermelon variety. As a consequence, according to this embodiment, because the first male flowers produced by a seeded pollenizer watermelon variety that matures in sufficient time to pollenize a seedless watermelon variety open prior to the first female flowers produced by said seedless watermelon variety, the male flowers of the seeded watermelon variety are able to pollenize the female flowers of the seedless watermelon variety. In other embodiments, the first male flowers produced by a seeded pollenizer watermelon variety that matures in sufficient time to pollenizer the female flowers of a seedless watermelon variety can open at about the same time on average as the opening of the first female flowers produced by said seedless watermelon variety, and thus the male flowers of the seeded watermelon variety are able to pollenize the female flowers of the seedless watermelon variety.

As is known in the art of watermelon breeding and cultivation, and as described herein, watermelon varieties can differ in their rate of maturation and the timing of the production of their flowers and fruit. Varieties that do vary in fruit maturation times can be referred to as an "early season", a "mid-season" or a "late season" variety. As would be appreciated by the skilled artisan, there is expected to be overlap in the production of flowers and the maturation of fruit between early, mid- and/or late season varieties. However, because of the variation in the opening of the first flowers, there is then variation in the timing of the production of the fruits. In representative embodiments, by providing this variation among a group of watermelon seedlings, both seeded and seedless varieties, in a single container, optionally wherein all varieties including the seeded pollenizer variety produce fruit suitable for consumption, the gardener can achieve the production of a variety of watermelon fruits, optionally seeded and seedless, for consumption over an extended season.

Accordingly, as used herein, an "early season" variety means that the first fruits of said variety are generally produced prior to (*i.e.,* earlier than; *e.g.,* on average about 3 days to about 12 days (*e.g*., about 3, 4, 5, 6, 7, 8, 9, 10, 11, 12 days and the like) before, and any value or range therein) the production of the first fruits by a mid-season variety. A "mid-season" variety refers to a watermelon variety that produces its first fruits earlier than (*e.g*., earlier than, for example, on average about 3 days to about 12 days before, and any range therein) the production of the first fruits by a "late season" variety. A "late season variety" refers to a watermelon variety in which the first fruits are generally produced after (*e.g*., later than; for example, on average, about 3 days to about 12 days later, and any range therein) the production of the first fruits by an "mid-season" variety, or after (*e.g.,* later than; for example, on average about 6 days to about 25 days (e.g., about 6, 7, 8, 9, 10, 11, 12, 13, 14, 15, 16, 17, 18, 19, 20, 21, 22, 23, 24, 25 days and the like) later, and any value or range therein) the production of the first fruits by a "early-season" variety. Therefore, the fruits of an early season variety are produced on average about 3 days to about 12 days prior to the production of the first fruits by a mid-season variety and the first fruits of a mid-season variety are produced on average about 3 days to about 12 days prior to the production of the first fruits of a late season variety, and the like. Thus, in some embodiments, an early season variety can produce its fruits on average at least about 3 days before the production of the first fruits of a mid-season variety and/or a late season variety. In other embodiments, a mid-season variety can produce its first fruits on average at least about 3 days before the production of the first fruits of a late season variety and/or on average at least about 3 days after the production of the first fruits of an early season variety. In still other embodiments, a late season variety can produce its first fruits on average at least about 3 days after the first fruits of a mid-season variety and/or an early season variety. In further embodiments, a late season variety can produce its first fruits on average at least about 3 days after the production of the first fruits of a mid-season variety and/or at least about 6 days after the production of the first fruits of an early season variety, and an early season variety can produce its first fruits on average at least about 3 days before the production of the first fruits of a mid-season variety and/or on average at least about 6 days before the production of the first fruits of a late season variety.

Thus, in some embodiments, at least one seedling of seeded pollenizer watermelon variety can mature (*e.g.,* produce its first flowers or its first fruit) on average at least about 3 days to about 25 days prior to a seedless watermelon variety that it pollinizes. Alternatively, at least one vegetatively-propagated watermelon variety can mature (*e.g.,* produce its first flowers or its first fruit) on average at least about 3 days to about 25 days prior to a seedless watermelon variety that it pollinizes.

As used herein, the term "pollenizer" refers to a plant that produces the male flowers, which are used as the source of pollen for pollinating female flower parts.

The present invention is directed to providing the grower (*e.g.,* home gardener, chef, and the like) with a solution to producing seedless watermelon fruits, optionally over an extended season. In some aspects of the invention, the containers of this invention can be prepared by a professional, commercial grower and then offered at retail stores to the grower, such as a home gardener or a chef wishing to produce seedless watermelons.

Accordingly, in one aspect of the invention, a single container comprising at least one seedling (*e.g.,* one, two, three, four, five, and the like) of a seedless watermelon variety and at least one seedling (*e.g.,* one, two, three, four, five, and the like) of a seeded pollenizer watermelon variety is provided. In a further aspect, a single container comprising at least two seedlings of a seedless watermelon variety and at least one seedling of a seeded pollenizer watermelon variety is provided. In other aspects, the at least two seedlings of a seedless watermelon variety can comprise, consist essentially of, or consist of a first and a second seedling from the same seedless watermelon variety. In a further aspect of the invention, the at least two seedlings of a seedless watermelon variety can comprise, consist essentially of, or consist of a first seedling from a first seedless watermelon variety and a first seedling from a second seedless watermelon variety. In some embodiments, the at least two seedlings of a seedless watermelon variety can comprise two or more varieties, wherein multiples (*e.g.,* two or more) of some or all of the two or more varieties are present. In some embodiments of the invention, the at least one seedling of a seeded pollenizer watermelon variety can comprise, consist essentially of, or consist of one seedling. In other embodiments, the at least one seedling of a seeded pollenizer watermelon variety can comprise, consist essentially of, or consist of a first and a second seedling from the same seeded pollenizer watermelon variety. In still other embodiments, the at least one seedling of a seeded pollenizer watermelon variety can comprise, consist essentially of, or consist of a first seedling from a first seeded pollenizer watermelon variety and a first seedling from a second seeded pollenizer watermelon variety. In embodiments, the at least two seedlings of a seeded pollenizer watermelon variety can comprise two or more varieties, wherein multiples (*e.g.,* two or more) of some or all of the two or more varieties are present.

In other aspects, a container of this invention comprises at least one seedling of a seedless watermelon variety and at least one seedling of a seeded pollenizer watermelon variety, wherein the at least one seedling of a seeded pollenizer watermelon variety produces male flowers in sufficient time to pollenize the female flowers of at least one seedling of a seedless watermelon variety. In further aspects, a container of this invention comprises at least one seedling of a seedless watermelon variety and at least one seedling of a seeded pollenizer watermelon variety, wherein the at least one seedling of a seeded pollenizer watermelon variety produces its first fruits prior to (*e.g.,* at least about 3, 4, 5, 6, 7, 8, 9, 10, 11, 12, 13, 14, 15, 16, 17, 18, 19, 20, 21, 22, 23, 24, 25 days before) the production of the first fruits of the at least one seedling of a seedless watermelon variety. In additional aspects of the invention, the at least one seedling of a seedless watermelon variety comprises, consists essentially of, or consists of one seedling and the at least one seedling of a seeded pollenizer watermelon variety comprises, consists essentially of, or consists of one seedling, wherein the one seedling of a seeded pollenizer variety produces male flowers in sufficient time to pollenize the female flowers of the one seeding of a seedless watermelon variety or produces its first fruits prior to the production of the first fruits of the one seedling of a seedless watermelon variety. Thus, in some embodiments, the at least one seedling of a seeded pollenizer watermelon variety can be an early season variety and the at least one seedling of a the seedless watermelon variety can be an early season variety, a mid-season variety or a late season variety (*e.g.,* the seeded pollenizer variety matures to produce its first fruits at about the same time or on average at least about 3 days to about 12 days prior to the production of the first fruits of the at least one seedling of a seedless watermelon variety). In still other embodiments, the at least one seedling of a seeded pollenizer watermelon variety can be a mid-season variety and the at least one seedling of a seedless watermelon variety can be a mid-season variety or a late season variety (*e.g.,* the at least one seedling of a the seeded pollenizer variety matures to produce its fruits at about the same time or on average at least about 3 days to about 12 days prior to the production of the first fruits of the at least one seedling of a seedless watermelon variety).

In a further aspect, a single container comprising at least two seedlings (*e.g.,* two, three, four, five, and the like) of a seedless watermelon variety and at least one seedling (*e.g.,* one, two, three, four, five, and the like) of a seeded pollenizer watermelon variety is provided, wherein the at least one seedling of a seeded pollenizer watermelon variety produces male flowers in sufficient time to pollenize the female flowers of the at least two seedlings of a seedless watermelon variety. In other aspects, a single container comprising at least two seedlings of a seedless watermelon variety and at least one seedling of a seeded pollenizer watermelon variety is provided, wherein the at least two seedlings of a seedless watermelon variety comprise, consist essentially of, or consist of a first seedling of a first seedless watermelon variety and a first seedling of a second seedless watermelon variety, and the at least one seedling of a seeded pollenizer watermelon variety produces male flowers in sufficient time to pollenize the female flowers of the first seedling of a first seedless watermelon variety and the first seedling of a second seedless watermelon variety. In representative embodiments, the at least one seedling of a the seeded pollenizer watermelon variety is an early season variety, the first seedling of a first seedless watermelon variety is a mid-season variety and the first seedling of a second seedless watermelon variety is a late season variety (*e.g.,* the first fruits of the early season seeded pollenizer variety are produced on average about 3 days to about 12 days prior to the production of the first fruits by the mid-season seedless variety and the first fruits of the mid-season seedless variety are produced on average about 3 days to about 12 days prior to the production of the first fruits of the late season seedless variety). In other embodiments, the first seedling of a seeded watermelon variety is an early season variety, and the first seedling of a first seedless watermelon variety and the first seedling of a second seedless watermelon variety are early season varieties and/or mid-season varieties (*e.g.,* the first and second seedless watermelon varieties can be the same variety or different varieties having the same or different times of production of first fruits). In still other embodiments, the first seedling of a seeded watermelon variety is a mid-season variety, and the first seedling of a first seedless watermelon variety and the first seedling of a second seedless watermelon variety are mid-season varieties and/or late season varieties (*e.g.,* the first and second seedless watermelon varieties can be the same variety or different varieties having the same or different times of production of first fruits). In further embodiments, the first seedling of a seeded watermelon variety is a mid-season variety, the first seedling of a first seedless watermelon variety is a mid-season variety and the first seedling of a second seedless watermelon variety is a late season variety.

In additional aspects, a single container comprising at least two seedlings of a seedless watermelon variety and at least one seedling of a seeded pollenizer watermelon variety is provided, wherein the at least two seedlings of a seedless watermelon variety comprise, consist essentially of, or consist of a first and a second seedling of the same seedless watermelon variety, the at least one seedling of a seeded pollenizer watermelon variety comprises, consists essentially of, or consists of a first and a second seedling of the same seeded pollenizer watermelon variety, and the first and second seedlings of a seeded pollenizer watermelon variety produce male flowers in sufficient time to pollenize the female flowers of the first and second seedlings of a first seedless watermelon variety. In representative embodiments, the first and second seedlings of a seeded watermelon variety can be an early season variety and the first and second seedlings of a seedless watermelon variety can be an early season variety, a mid-season variety or a late season variety. In other embodiments, the first and second seedlings of a seeded watermelon variety can be a mid-season variety, and the first and second seedlings of a seedless watermelon variety can be a mid-season variety or a late season variety.

In additional aspects, a single container comprising at least two seedlings of a seedless watermelon variety and at least one seedling of a seeded pollenizer watermelon variety is provided, wherein the at least two seedlings of a seedless watermelon variety comprise, consist essentially of, or consist of a first seedling of a first seedless watermelon variety and a first seedling of a second seedless watermelon variety, the at least one seedling of a seeded pollenizer watermelon variety comprises, consists essentially of, or consists of a first and a second seedling of the same seeded pollenizer watermelon variety, and the first and a second seedlings of a seeded pollenizer watermelon variety produce male flowers in sufficient time to pollenize the female flowers of the first seedling of a first seedless watermelon variety and the female flowers of the first seedling of a second seedless watermelon variety. In representative embodiments, the seeded watermelon variety can be an early season variety, the first seedless watermelon variety can be a mid-season variety and the second seedless watermelon variety can be a mid-season variety or a late season variety. In other embodiments, the seeded watermelon variety can be an early season variety, and the first seedless watermelon variety and the second seedless watermelon variety can be early season varieties and/or mid-season varieties. In still other embodiments, the seeded watermelon variety can be a mid-season variety, and the first seedless watermelon variety and the second seedless watermelon variety can be mid-season varieties and/or late season varieties. In representative embodiments, the seeded watermelon variety can be a mid-season variety, the first seedless watermelon variety can be a mid-season variety and the second seedless watermelon variety can be a late season variety.

In additional aspects, a single container comprising at least two seedlings of a seedless watermelon variety and at least one seedling of a seeded pollenizer watermelon variety is provided, wherein the at least two seedlings of a seedless watermelon variety comprise, consist essentially of, or consist of a first seedling of a first seedless watermelon variety and a second seedling of a second seedless watermelon variety, the at least one seedling of a seeded pollenizer watermelon variety comprises, consists essentially of, or consists of a first seedling of a first seeded pollenizer watermelon variety and a second seedling of a second seeded pollenizer watermelon variety, and the first seedling of a first seeded pollenizer watermelon variety and the second seedling of a second seeded pollenizer watermelon variety each produce male flowers in sufficient time to pollenize the female flowers of the at least one of the first seedling of a first seedless watermelon variety and/or the female flowers of the second seedling of a second seedless watermelon variety. Accordingly, the first seedling of a first seeded pollenizer watermelon variety can produce male flowers in sufficient time to pollenize the female flowers of both or one of the first seedling of a first seeded pollenizer watermelon variety and/or the second seedling of a second seeded pollenizer watermelon variety, and the second seedling of a second seeded pollenizer watermelon variety can produce male flowers in sufficient time to pollenize the female flowers of both or one of the first seedling of a first seeded pollenizer watermelon variety and/or the second seedling of a second seeded pollenizer watermelon variety. In some embodiments, the first and the second seeded watermelon varieties can both be early season varieties, the first seedless watermelon variety can be a mid-season variety and the second seedless watermelon variety can be a late season variety. In other embodiments, the first and second seeded watermelon varieties can be early season varieties, and the first seedless watermelon variety and the second seedless watermelon variety can be early season varieties, mid-season varieties and/or late season varieties. In still other embodiments, the first seeded watermelon variety can be an early season variety, the first seedless watermelon variety and the second seeded pollenizer watermelon variety can be mid-season varieties and the second seedless watermelon variety can be a late season variety. In further embodiments, the first seeded watermelon variety can be an early season variety, the second seeded pollenizer watermelon variety can be a mid-season variety and the first seedless watermelon variety and the second seedless watermelon variety can be late season varieties. In still further embodiments, the first seeded watermelon variety can be an early season variety, the second seeded pollenizer watermelon variety can be a mid-season variety and the first seedless watermelon variety can be an early season or a mid-season variety and the second seedless watermelon variety can be an early season or mid-season variety.

In particular embodiments, a single container of this invention can comprise at least two watermelon plants (*e.g.,* two, three, four, five, six, seven, eight, nine, ten, and the like) with the total number of plants dependent on the size of the container. Further, not all possible combinations of early, mid- and late season seeded pollenizer and seedless watermelon varieties are specifically recited herein; however, the skilled horticulturalist is able to not only determine the total number of plants that is appropriate for any given container and container size but with the guidance of this invention is also able to determine the appropriate combination of seedless and seeded pollenizer varieties.

Alternatively, it would be clear to the skilled person that any of the above embodiments can similarly be practiced using vegetatively-propagated watermelon varieties, such as e.g. cuttings or plants derived from tissue or cell culture. In a particular embodiment, a container can comprise at least two vegetatively-propagated materials of at least one seedless watermelon variety and at least one vegetatively-propagated material of a seeded pollenizer watermelon variety. In a further embodiment, the two vegetatively-propagated materials of at least one seedless watermelon variety are from the same seedless watermelon variety or at least two different seedless watermelon varieties. In a still further embodiment, the at least two vegetatively-propagated materials of at least one seedless watermelon variety consists of a first and a second vegetatively-propagated material from the same seedless watermelon variety or different seedless watermelon varieties. In a still further embodiment, the at least one vegetatively-propagated material of a seeded pollenizer watermelon variety consists of one vegetatively-propagated material or a first and a second vegetatively-propagated material from the same seeded pollenizer watermelon variety or different seeded pollenizer watermelon varieties. In a still further embodiment, the at least one vegetatively-propagated material of a seeded pollenizer watermelon variety consists of a first and a second vegetatively-propagated material from the same seeded pollenizer watermelon variety. In a still further embodiment, the at least one vegetatively-propagated material of a seeded pollenizer watermelon variety produces male flowers in sufficient time to pollinize female flowers of the at least two vegetatively-propagated materials of at least one seedless watermelon variety. In a still further embodiment, the first and second vegetatively-propagated materials of a seeded pollenizer watermelon variety produce male flowers in sufficient time to pollinize female flowers of the first and second vegetatively-propagated materials of the seedless watermelon variety(ies). In a still further embodiment, the at least one vegetatively-propagated material of a seeded pollenizer watermelon variety is an early season variety, the first vegetatively-propagated material of a seedless watermelon variety is a mid-season variety, and the second vegetatively-propagated material of a seedless watermelon variety is a late season variety. In a still further embodiment, the seeded pollenizer watermelon variety is an early season variety and the first and second vegetatively-propagated materials of a seedless watermelon variety are a mid-season variety and a late season variety, respectively. In a still further embodiment, the at least one vegetatively-propagated material of a seeded pollenizer watermelon variety is an early season variety and the first and second vegetatively-propagated materials of a seedless watermelon variety are both the same mid-season variety or the same late season variety. In a still further embodiment, the at least one vegetatively-propagated material of a seeded pollenizer watermelon variety is a first and second vegetatively-propagated material.

In another embodiment, the container comprises at least one vegetatively-propagated material of a seedless watermelon variety and at least one vegetatively-propagated material of a seeded pollenizer watermelon variety, wherein the at least one vegetatively-propagated material of a seeded pollenizer watermelon variety produces its first fruits prior to the production of the first fruits of the at least one vegetatively-propagated material of a seedless watermelon variety, thereby providing watermelon fruits over an extended season. In a still further embodiment, the at least one vegetatively-propagated material of a seeded pollenizer watermelon variety is an early season variety and the at least one vegetatively-propagated material of a seedless watermelon variety is a mid-season variety or a late season variety.

The present invention provides methods for producing watermelon fruits, optionally over an extended season, comprising providing a container of this invention as described herein.

The invention also encompasses methods for producing watermelon fruit *e.g*., in the garden, *e.g*., in the home garden, optionally over an extended season are provided comprising planting together in a single container a seedless watermelon variety and a seeded pollenizer watermelon variety having any of the configurations described herein, optionally to produce watermelon fruit over an extended period. In some embodiments, methods for producing watermelon fruit *e.g.,* in the garden, *e.g.,* in the home garden, optionally over an extended season are provided, comprising planting together in a single container at least one seedling (*e.g*., one, two, three, four, five and the like) of a seedless watermelon variety and at least one seedling (*e.g*., one, two, three, four, five and the like) of a seeded pollenizer watermelon variety, wherein the at least one seedling of a seeded pollenizer watermelon variety produces male flowers in sufficient time to pollenize the female flowers of the at least one seedling of a seedless watermelon variety, thereby producing watermelon fruit, optionally over an extended period. In some embodiments of the invention, the at least one seedling of a seeded pollenizer watermelon variety can comprise, consist essentially of, or consist of one seedling or of two seedlings and the least one seedling of a seedless watermelon variety can comprise, consist essentially of, or consist of one seedling or of two seedlings, wherein when there are at least two seedlings of a seeded pollenizer watermelon variety, they can be of the same variety or of different varieties, and when there are at least two seedlings of a seedless watermelon variety, they can be of the same variety or of different varieties. Accordingly, in some aspects of the invention, the at least one seedling of a seedless watermelon variety comprises, consists essentially of, or consists of one seedling and the at least one seedling of a seeded pollenizer watermelon variety comprises, consists essentially of, or consists of one seedling, wherein the at least one seedling of a seeded pollenizer variety produces male flowers in sufficient time to pollenize the female flowers of the at least one seedling of a seedless watermelon variety. Thus, in some embodiments, the at least one seedling of a seeded pollenizer watermelon variety can be an early season variety and the at least one seedling of a seedless watermelon variety can be an early season, a mid-season or a late season variety. In still other embodiments, the at least one seedling of a seeded pollenizer watermelon variety can be a mid-season variety and the at least one seedling of a seedless watermelon variety can be a mid-season variety or a late season variety.

In additional embodiments, a method for producing watermelon fruit (*e.g.,* in the home garden), optionally over an extended season, is provided, the method comprising, consisting essentially of, or consisting of planting together in a single container at least two seedlings of a seedless watermelon variety and at least one seedling of a seeded pollenizer watermelon variety, wherein the at least one seedling of a seeded pollenizer watermelon variety produces male flowers in sufficient time to pollenize the female flowers of the at least two seedlings of a seedless watermelon variety. In some embodiments of the invention, the at least two seedlings of a seedless watermelon variety comprise, consist essentially of, or consist of a first seedling of a first seedless watermelon variety and a first seedling of a second seedless watermelon variety and the at least one seedling of a seeded pollenizer watermelon variety produces male flowers in sufficient time to pollenize the female flowers of the first seedling of a first seedless watermelon variety and the female flowers of the first seedling of a second seedless watermelon variety. In representative embodiments, the at least one seedling of a seeded watermelon variety is an early season variety, the first seedling of a first seedless watermelon variety is a mid-season variety and the first seedling of a second seedless watermelon variety is a late season variety. In other embodiments, the at least one seedling of a seeded watermelon variety is an early season variety, and the at least one seedling of a first seedless watermelon variety and the at least one seedling of a second seedless watermelon variety are early season varieties, mid-season varieties and/or late season varieties. In still other embodiments, the at least one seedling of a seeded watermelon variety is a mid-season variety, and the at least one seedling of a first seedless watermelon variety and the at least one seedling of a second seedless watermelon variety are mid-season varieties and/or late season varieties. In representative embodiments, the at least one seedling of a seeded watermelon variety is a mid-season variety, the at least one seedling of a first seedless watermelon variety is a mid-season variety and the at least one seedling of a second seedless watermelon variety is a late season variety. In yet further embodiments, the at least one seedling of a seeded watermelon variety is an early season variety, the at least one seedling of a first seedless watermelon variety is an early season variety and the at least one seedling of a second seedless watermelon variety is a mid-season variety or a late season variety.

In additional aspects, a method for producing watermelon fruit, optionally over an extended season, is provided, the method comprising, consisting essentially of, or consisting of planting together in a single container at least two seedlings of a seedless watermelon variety and at least one seedling of a seeded pollenizer watermelon variety, wherein the at least two seedlings of a seedless watermelon variety comprise, consist essentially of, or consist of a first and a second seedling of the same seedless watermelon variety, the at least one seedling of a seeded pollenizer watermelon variety comprises, consists essentially of, or consists of a first and a second seedling of the same seeded pollenizer watermelon variety, and the first and second seedlings of a seeded pollenizer watermelon variety produce male flowers in sufficient time to pollenize the female flowers of the first and the second seedlings of a first seedless watermelon variety. In representative embodiments, the at least one seedling of a seeded watermelon variety can be an early season variety and the first and a second seedlings of a seedless watermelon variety can be an early season variety, a mid-season variety or a late season variety. In other embodiments, the at least one seedling of a seeded watermelon variety can be a mid-season variety, and the first and a second seedlings of a seedless watermelon variety can be a mid-season variety or a late season variety.

In additional aspects, a method for producing watermelon fruit, optionally over an extended season, is provided, the method comprising, consisting essentially of, or consisting of planting together in a single container at least two seedlings of a seedless watermelon variety and at least one seedling of a seeded pollenizer watermelon variety, wherein the at least two seedlings of a seedless watermelon variety comprise, consist essentially of, or consist of a first seedling of a first seedless watermelon variety and a first seedling of a second seedless watermelon variety, the at least one seedling of a seeded pollenizer watermelon variety comprises, consists essentially of, or consists of a first and a second seedling of the same seeded pollenizer watermelon variety and the first and a second seedlings of a seeded pollenizer watermelon variety produce male flowers in sufficient time to pollenize the female flowers of the first seedling of a first seedless watermelon variety and of the first seedling of a second seedless watermelon variety. In representative embodiments, the first and second seedlings of a seeded watermelon variety can be an early season variety, the first seedling of a seedless watermelon variety can be a mid-season variety and the first seedling of a second seedless watermelon variety can be a late season variety. In other embodiments, the first and second seedlings of a seeded watermelon variety can be an early season variety, and the first seedling of a first seedless watermelon variety and the first seedling of a second seedless watermelon variety can be early season varieties, mid- season varieties and/or late season varieties. In still other embodiments, the first and second seedlings of a seeded watermelon variety can be a mid-season variety, and the first seedling of a first seedless watermelon variety and the first seedling of a second seedless watermelon variety can be mid-season varieties and/or late season varieties. In representative embodiments, the first and second seedlings of a seeded watermelon variety can be a mid-season variety, the first seedling of a first seedless watermelon variety can be a mid-season variety and the first seedling of a second seedless watermelon variety can be a late season variety.

In additional aspects, a method for producing watermelon fruit in the home garden over an extended season is provided, the method comprising, consisting essentially of, or consisting of planting together in a single container at least two seedlings of a seedless watermelon variety and at least one seedling of a seeded pollenizer watermelon variety, wherein the at least two seedlings of a seedless watermelon variety comprise, consist essentially of, or consist of a first seedling of a first seedless watermelon variety and a first seedling of a second seedless watermelon variety, the at least one seedling of a seeded pollenizer watermelon variety comprises, consists essentially of, or consists of a first seedling of a first seeded pollenizer watermelon variety and a first seedling of a second seeded pollenizer watermelon variety and the first seedling of a first seeded pollenizer watermelon variety and the second seedling of a second seeded pollenizer watermelon variety each produce male flowers in sufficient time to pollenize the female flowers of at least one of the first seedling of a first seedless watermelon variety and/or of the first seedling of a second seedless watermelon variety. Accordingly, the first seedling of a first seeded pollenizer watermelon variety can produce male flowers in sufficient time to pollenize the female flowers of one or both of the first seedling of a first seeded pollenizer watermelon variety and/or the second seedling of a second seeded pollenizer watermelon variety and the second seedling of a second seeded pollenizer watermelon variety can produce male flowers in sufficient time to pollenizer the female flowers of one or both of the first seedling of a first seeded pollenizer watermelon variety and/or the second seedling of a second seeded pollenizer watermelon variety. In some embodiments, the first and second seeded watermelon varieties can both be early season varieties, and the first seedless watermelon variety and the second seedless watermelon variety can be early season varieties, mid-season varieties and/or late season varieties. In other embodiments, the first and second seeded watermelon varieties can be early season varieties and/or mid-season varieties, and the first seedless watermelon variety and the second seedless watermelon variety can be mid-season varieties and/or late season varieties. In representative embodiments, the first and second seeded watermelon varieties can both be early season varieties, the first seedless watermelon variety can be a mid-season variety and the second seedless watermelon variety can be a late season variety. In further embodiments, the first seeded watermelon variety can be an early season variety, the first seedless watermelon variety and the second seeded pollenizer watermelon variety can be mid-season varieties and the second seedless watermelon variety can be a late season variety. In still further embodiments, the first seeded watermelon variety can be an early season variety, the second seeded pollenizer watermelon variety can be a mid-season variety and the first seedless watermelon variety and the second seedless watermelon variety can be late season varieties.

As described herein, in representative embodiments, this invention provides methods using seeded pollenizer watermelon varieties and seedless watermelon varieties that can have different times to maturity and thus, different times to flower and fruit production. According to these embodiments, by providing seeded and seedless watermelon varieties in a single container having staggered times to maturity, flowering and fruiting, a gardener can achieve the production of edible watermelon fruits (optionally, seeded and seedless) over an extended season.

As used herein, "extended season" means that the watermelon fruit production extends from or spans early season through mid-season and/or late season, or mid-season through late season.

As is apparent from the descriptions of the containers and methods for producing watermelon fruit provided herein, many different combinations are possible. Thus, as taught herein, not only can the seedless and seeded pollenizer watermelon varieties that are provided in a single container be varied but the total seedling number per container can be varied. Using the guidance of the present invention, the skilled horticulturalist is capable of determining the appropriate number of seedlings and arrangement of seeded pollenizer and seedless varieties to combine in any single container for producing watermelon fruit over an extended season. **Table 1** below provides some exemplary combinations of two to four seedlings that can be planted together in a single container.

| **Table 1.** Exemplary combinations of seeded and seedless watermelon varieties for use in containers and methods of this invention | | | | |
|---|---|---|---|---|
| | Seeded Watermelon | | Seedless Watermelon | |
| | Variety A (early season) | Variety B (mid-season) | Variety C (mid-season) | Variety D (late season) |
| Number of Seedlings | 1 | | 1 | |
| | | 1 | | 1 |
| | 1 | | 2 | |
| | | 1 | | 2 |
| | 1 | | 1 | 1 |
| | | 1 | 1 | 1 |
| | 2 | | 2 | |
| | | 2 | | 2 |
| | 2 | | 1 | 1 |
| | | 2 | 1 | 1 |
| | 1 | 1 | 2 | |
| | 1 | 1 | | 2 |
| | 1 | 1 | 1 | 1 |

As would be understood by the skilled artisan, additional useful combinations of five or more seedlings of seeded pollenizer and seedless watermelon varieties are encompassed by this invention.

Alternatively, it would be clear to the skilled person that any of the above methods and combinations can also be practiced using vegetatively-propagated watermelon varieties, such as *e.g.* cuttings or plants derived from tissue or cell culture.

In a particular embodiment, is disclosed a method for producing watermelon fruit over an extended season, comprising providing a container which comprises at least two vegetatively-propagated materials of at least one seedless watermelon variety and at least one vegetatively-propagated material of a seeded pollenizer watermelon variety. In a still further embodiment is disclosed a method for producing watermelon fruit over an extended season, comprising
planting together in a container at least one vegetatively-propagated material of a seedless watermelon variety and at least one vegetatively-propagated material of a seeded pollenizer watermelon variety, wherein the at least one vegetatively-propagated material of a seeded pollenizer watermelon variety matures in sufficient time to pollinize the at least one vegetatively-propagated material of a seedless watermelon variety, thereby producing watermelon fruit over an extended period. In a still further embodiment is disclosed a method further comprising transplanting the vegetatively-propagated materials into the ground or into a larger container, wherein the vegetatively-propagated materials planted in the container form a single root ball and are removed from the single container and planted together into the ground or into a larger container without substantially disrupting said root ball. In a still further embodiment is disclosed a method wherein the at least one vegetatively-propagated material of a seedless watermelon variety comprises at least two vegetatively-propagated materials of at least one seedless watermelon variety. In a still further embodiment is disclosed a method, wherein the at least one vegetatively-propagated material of a seedless watermelon variety consists of a first vegetatively-propagated material of a first seedless watermelon variety and a first vegetatively-propagated material of a second seedless watermelon variety. In a still further embodiment is disclosed a method, wherein the at least one vegetatively-propagated material of a seeded pollenizer watermelon variety consists of a first and a second vegetatively-propagated material of the same seeded pollenizer watermelon variety. In a still further embodiment is disclosed a method, wherein the at least one vegetatively-propagated material of the seeded pollenizer watermelon variety produces male flowers in sufficient time to pollinize female flowers of the at least two vegetatively-propagated materials of at least one seedless watermelon variety. In a still further embodiment is disclosed a method, wherein the first and the second vegetatively-propagated materials of the same seeded pollenizer watermelon variety produce male flowers in sufficient time to pollinize female flowers of the first vegetatively-propagated material of a first seedless watermelon variety and female flowers of the first vegetatively-propagated material of a second seedless watermelon variety. In a still further embodiment is disclosed a method, wherein the seeded pollenizer watermelon variety is an early season variety and the seedless watermelon variety is a mid-season variety or a late season variety. In a still further embodiment is disclosed a method, wherein the seeded pollenizer watermelon variety is an early season variety, the first seedless watermelon variety is a mid-season variety and the second seedless watermelon variety is a late season variety.

Any seedless watermelon variety that can be pollinated with the pollen of a selected seeded pollenizer watermelon variety can be used with this invention. Seeded pollenizer watermelon varieties useful with this invention can include any seeded watermelon variety, the plants of which mature in sufficient time to pollenize the plants of the seedless watermelon variety(ies) planted in the same container as described and claimed herein, and optionally produce edible fruit. Thus, the seeded pollenizer watermelon varieties useful with this invention not only pollenize the seedless watermelon varieties but self pollinate so that both seeded and seedless edible watermelon fruits are produced. Consequently, the seeded pollenizer watermelon varieties used in today's commercial setting are typically not useful with this invention. These varieties are often referred to as "dedicated pollenizers" or "non-harvestable pollinizers" (*e.g*., SP-1, SP-4, Sidekick, Patron) and they are bred to produce small, brittle, non-harvestable fruit. These fruit are bitter tasting or do have very low sugar content and would generally be considered non-edible by the average consumer. Accordingly, in embodiments, the seeded pollenizer variety is not an enhanced watermelon pollenizer. In contrast, in embodiments of the present invention, the seeded pollenizer watermelon variety that is provided in a container with seedless watermelon varieties is self-pollinating to provide a gardener with edible seeded fruit.

Non-limiting examples of seeded pollenizer varieties include 'Quetzali', 'Sangria', 'Top Gun', 'Jamboree', 'Estrella', 'Mardi Gras' and 'Sunsugar'. Nonlimiting examples of seedless watermelon varieties useful with this invention include 'Sweet Gem', 'Dorin' ('RWT8212'), 'Amarillo', 'Imagination', 'Fascination', 'Tri-X® Brand 313', 'Yellow Bite', 'Melody' and 'Exclamation'. 'Quetzali' is an example of an early season commercial seeded pollenizer watermelon variety capable of providing a pollen source for seedless watermelon varieties as described herein. 'Top Gun', 'Sunsugar', 'Estrella' and 'Mardi Gras' are examples of mid-season seeded pollenizer watermelon varieties and 'Sangria' and 'Jamboree' are examples of late season seeded pollenizer watermelon varieties. 'Sweet Gem' is a dark rind, early to mid-season commercial seedless watermelon variety and 'Dorin' ('RWT82812') is another early season commercial seedless watermelon that is small with a dark rind. 'Yellow Bite' is an early season personal sized seedless watermelon with yellow flesh, 'Melody' is another early season seedless variety. 'Amarillo' and 'Imagination' are two further examples of early to mid-season seedless watermelon varieties. 'Fascination' is an example of mid-season seedless watermelon variety and 'Tri-X® Brand 313' and 'Exclamation' are examples of late season seedless watermelon varieties. It is noted that these are just a few examples of varieties of seeded and seedless pollenizer watermelon varieties that are available. The skilled artisan in the art of watermelon breeding would be able to identify other varieties, known and not yet developed that would be satisfactory for use with this invention.

Thus, in particular embodiments, the at least one seedling or the first and the second seedling of at least one seeded pollenizer watermelon variety can be 'Quetzali', 'Sangria', 'Top Gun', 'Jamboree', 'Estrella', 'Mardi Gras' and/or 'Sunsugar' and the at least one seedling or the first and the second seedling of at least one of a seedless watermelon variety can be 'Sweet Gem', 'Dorin', 'Amarillo', 'Imagination', 'Fascination', 'Exclamation', 'Yellow Bite', 'Melody' and/or 'Tri-X® Brand 313'.

Accordingly, in some embodiments, a container of the present invention comprises, consists essentially of, or consists of a first and a second seedling of a seeded pollenizer watermelon variety and a first and a second seedling of a seedless watermelon variety, wherein the seeded pollenizer watermelon variety can be 'Quetzali', 'Sangria', 'Top Gun', 'Jamboree', 'Estrella', 'Mardi Gras' and/or 'Sunsugar' and the seedless watermelon variety can be 'Sweet Gem', 'Dorin', 'Amarillo', 'Imagination', 'Fascination', 'Tri-X® Brand 313', 'Yellow Bite', 'Melody' and/or 'Exclamation'. In further embodiments, a container of the present invention comprises, consists essentially of, or consists of a first and a second seedling of a seeded pollenizer watermelon variety and a first seedling of first seedless watermelon variety and a second seedling of second seedless watermelon variety, wherein the seeded pollenizer watermelon variety can be 'Quetzali', 'Sangria', 'Top Gun', 'Jamboree', 'Estrella', 'Mardi Gras' and/or 'Sunsugar' and the first seedless watermelon variety and the second seedless watermelon variety can be 'Sweet Gem', 'Dorin', 'Amarillo', 'Imagination', 'Fascination', 'Exclamation', 'Yellow Bite', 'Melody' and/or 'Tri-X® Brand 313'. In still further embodiments, a container of the present invention comprises, consists essentially of, or consists of a first seedling of a first seeded pollenizer watermelon variety, a second seedling of a second seeded pollenizer watermelon variety, a first seedling of first seedless watermelon variety and a second seedling of second seedless watermelon variety, wherein the first seeded pollenizer watermelon variety and the second seeded pollenizer watermelon variety can be can be 'Quetzali', 'Sangria', 'Top Gun', 'Jamboree', 'Estrella', 'Mardi Gras' and/or 'Sunsugar', the first seedless watermelon variety and the second seedless watermelon variety can be 'Sweet Gem', 'Dorin', 'Amarillo', 'Imagination', 'Fascination', 'Yellow Bite', 'Melody' and/or 'Tri-X® Brand 313'.

In representative embodiments, the seeded pollenizer watermelon variety is 'Quetzali', the first seedless watermelon variety is 'Sweet Gem', and the second seedless watermelon variety is 'Dorin', 'Melody' or "Yellow Bite'.

The methods of the present invention for producing watermelon fruit, optionally over an extended season, provide similar useful combinations of seeded pollenizer watermelon varieties and seedless watermelon varieties. Accordingly, in some embodiments, methods for producing watermelon fruit in the garden over an extended season are provided, comprising planting together in a single container at least one seedling of a seedless watermelon variety and at least one seedling of a seeded pollenizer watermelon variety, wherein the seeded pollenizer watermelon variety can be 'Quetzali', 'Sangria', 'Top Gun', 'Jamboree', 'Estrella', 'Mardi Gras' and/or 'Sunsugar' and the seedless watermelon variety can be 'Sweet Gem', 'Dorin', 'Amarillo', 'Imagination', 'Fascination', 'Exclamation', 'Yellow Bite', 'Melody' and/or 'Tri-X® Brand 313'. In other embodiments, a method for producing watermelon fruit in the garden over an extended season is provided, the method comprising, consisting essentially of, or consisting of planting together in a single container at least two seedlings of a seedless watermelon variety and at least one seedling of a seeded pollenizer watermelon variety, wherein the at least two seedlings of a seedless watermelon variety can be 'Sweet Gem', 'Dorin', 'Amarillo', 'Imagination', 'Fascination', 'Exclamation', 'Yellow Bite', 'Melody' and/or 'Tri-X® Brand 313' and the seeded pollenizer watermelon variety can be 'Quetzali', 'Sangria', 'Top Gun', 'Jamboree', 'Estrella', 'Mardi Gras' and/or 'Sunsugar'.

In further embodiments, a method for producing watermelon fruit in the garden over an extended season is provided, the method comprising, consisting essentially of, or consisting of planting together in a single container a first and a second seedling of a seeded pollenizer watermelon variety and a first seedling of first seedless watermelon variety and a second seedling of second seedless watermelon variety, wherein the seeded pollenizer watermelon variety can be 'Quetzali', 'Sangria', 'Top Gun', 'Jamboree', 'Estrella', 'Mardi Gras' or 'Sunsugar' and the first seedless watermelon variety can be 'Sweet Gem', 'Dorin', 'Amarillo', 'Imagination', 'Fascination', 'Exclamation', 'Yellow Bite', 'Melody' or 'Tri-X® Brand 313' and the second seedless watermelon variety can be 'Sweet Gem', 'Dorin', 'Amarillo', 'Imagination', 'Fascination', 'Yellow Bite', 'Melody' or 'Tri-X® Brand 313'. In still further embodiments, a method for producing watermelon fruit in the garden over an extended season is provided, the method comprising, consisting essentially of, or consisting of planting together in a single container a first seedling of a first seeded pollenizer watermelon variety, a second seedling of a second seeded pollenizer watermelon variety, a first seedling of first seedless watermelon variety and a second seedling of second seedless watermelon variety, wherein the first seeded pollenizer watermelon variety can be 'Quetzali' , 'Sangria' , 'Top Gun', 'Jamboree', 'Estrella', 'Mardi Gras' or 'Sunsugar,' the second seeded pollenizer watermelon variety can be Quetzali,' Sangria,' 'Top Gun', 'Jamboree,' 'Estrella', 'Mardi Gras' or 'Sunsugar,' the first seedless watermelon variety can be 'Sweet Gem,' 'Dorin,' 'Amarillo,' 'Imagination,' 'Fascination,' 'Exclamation', 'Yellow Bite', 'Melody' or 'Tri-X® Brand 313' and the second a seedless watermelon variety can be 'Sweet Gem', 'Dorin', 'Amarillo', 'Imagination', 'Fascination', 'Exclamation', 'Yellow Bite', 'Melody' or 'Tri-X® Brand 313'.

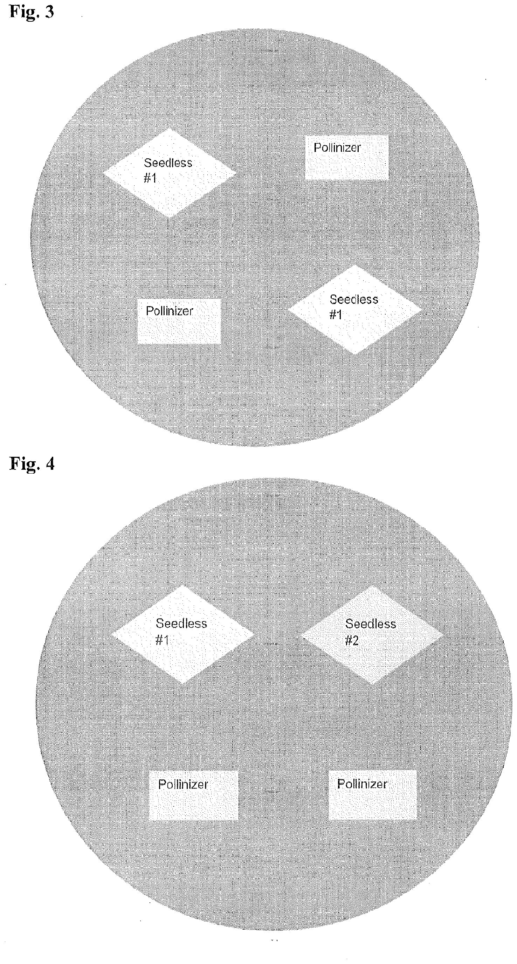

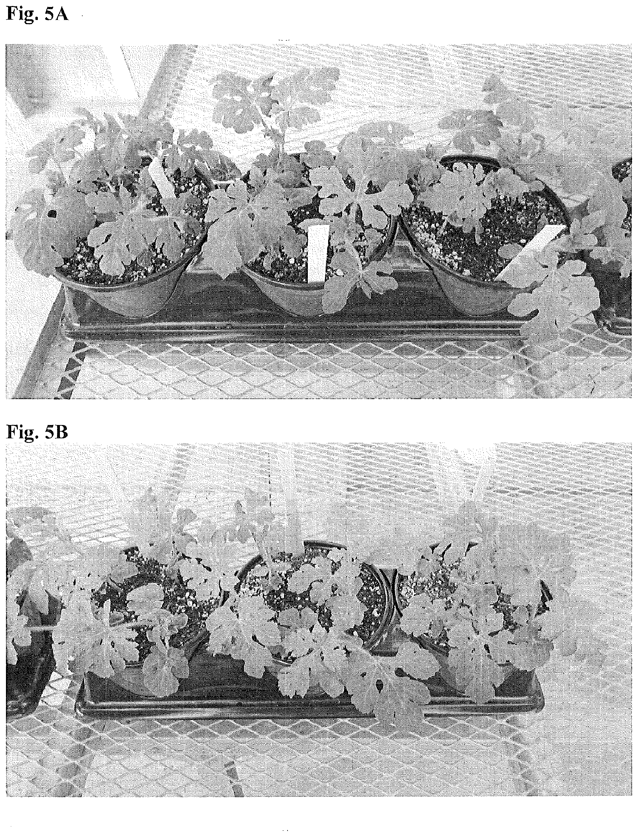

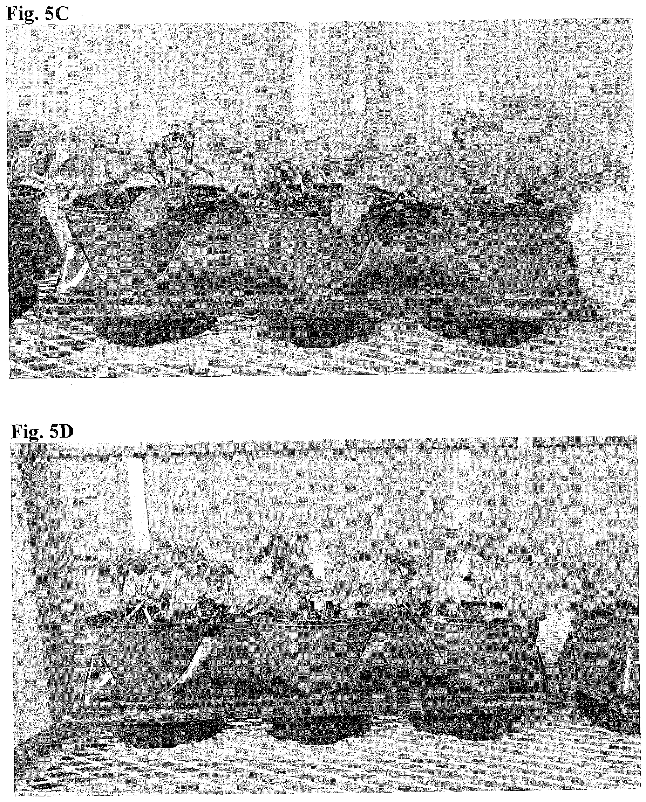

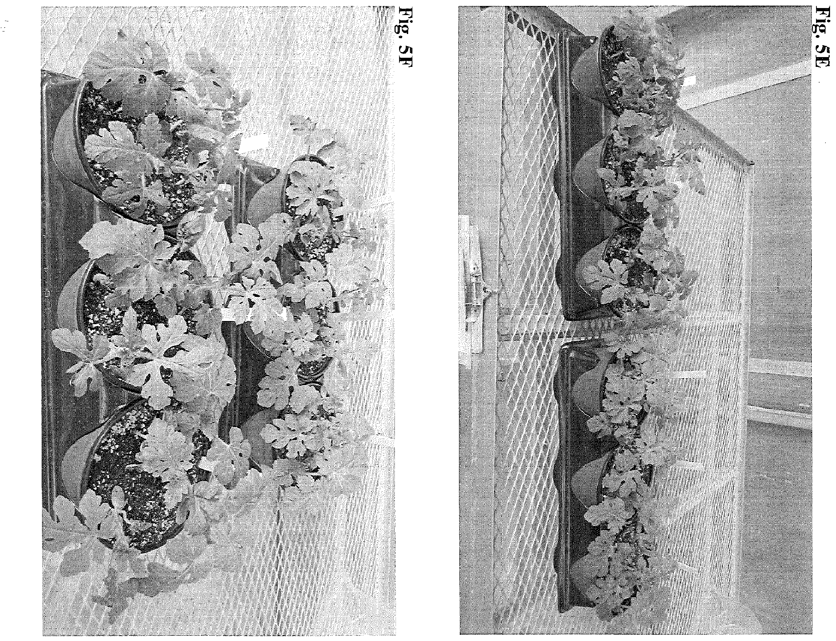

In particular aspects of the invention, the fruit produced by a seeded pollenizer watermelon variety(ies) can be visually distinguished from the fruit produced by a seedless watermelon variety(ies). Thus, in representative embodiments, the fruit produced by the at least one seedling of a seeded watermelon pollenizer variety can be visually distinguished from the fruit produced by the at least one seedling of a seedless watermelon variety, or by the at least two seedlings of a seedless watermelon variety (see, *e.g*., **Fig. 4A-4C** and **Fig. 5A-5F**). In further embodiments, the fruit produced by a first seedling from a first seedless watermelon variety can be visually distinguished from the fruit produced by a second seedling from a second seedless watermelon variety, and the fruit produced by each of the first and second seedlings of seedless watermelon varieties can be visually distinguished from the seeded watermelon variety. "Visually distinguishable" features can include size, color and/or shape, for example a solid colored rind versus a striped rind or any characteristic that allows the varieties to be distinguished.

Alternatively, the fruit of the at least one vegetatively-propagated material of the seeded pollenizer watermelon variety is visually distinguishable from the fruit of the first and second vegetatively-propagated materials of the seedless watermelon varieties. In a further embodiment, the seeded pollenizer watermelon variety is 'Quetzali', 'Sangria', 'Top Gun', 'Jamboree', 'Estrella', 'Mardi Gras' and/or 'Sunsugar' and the at least one seedless watermelon variety is 'Sweet Gem', 'Dorin', 'Amarillo', 'Imagination', 'Fascination', 'Yellow Bite', 'Melody' and/or 'Tri-X® Brand 313'. In a still further embodiment, the seeded pollenizer watermelon variety is 'Quetzali,' and the first vegetatively-propagated material of a seedless watermelon variety is 'Sweet Gem', and the second vegetatively-propagated material of a seedless watermelon variety is 'Dorin', 'Melody' or 'Yellow Bite'.

In another aspect of the invention is disclosed the use of a container according to any of the embodiments for the production of watermelon fruit over an extended period.

Seedlings can be produced using any suitable method in the art. For example, prior to being placed together in a single container together, seeds of the seedless watermelon variety(ies) and seeds of the seeded pollenizer watermelon variety(ies) are typically first sown into, germinated and grown separately in, for example, plug trays. Some common trays used for growing watermelon seedlings range from 98, 128, 200 and 242 cells per tray. Such "plug" or "cell" trays can be, for example, about 30 to 50 cm³ in size. The trays are composed of various materials including but not limited to hard styrofoam, hard plastic or flexible plastic materials.

Alternatively, in some embodiments, seeds of the seeded pollenizer variety(ies) and the seedless watermelon variety(ies) can be sown directly into the container rather than being transplanted as seedlings into the single container. When directly sowing the seeds into the container, the methods for making the containers of this invention are otherwise the same as set forth herein.

In general, seedlings are ready for transplanting into a single container as described herein when the roots are sufficiently developed to permit removal from the cell with the growing media volume substantially intact, generally about four to about seven weeks (*e.g.,* four weeks, five weeks, six weeks, seven weeks, and the like, and any range therein) from sowing or seeding, depending on cell size, light and temperature conditions.

The seedlings that are initially germinated and grown for sufficient time (*i.e.,* roots are sufficiently developed to permit removal from the cell with the growing media volume substantially intact) in individual containers can then be planted together in a single container comprising growth media as described herein.

Seedlings (or seeds) can be planted in groups totaling about 2, 3, 4, 5, 6, 7, 8, 9, 10 or more seedlings (or about 2, 3, 4, 5, 6, 7, 8, 9, 10 or more seeds), and the like, depending on the size of the single container used. "Growth media" as used herein means any media in which a watermelon seedling can be grown and established. Non-limiting examples of growth media include potting soil, perlite, vermiculite, sphagnum, peat moss, clay, white pied, and combinations thereof. Of course, in addition to the growth media, water and optional nutrients can be added. The seedlings (transplanted or directly sown as seeds) in the single container are then grown for an additional about two to three weeks to allow the seedlings to root out the soil mass (to form a root ball comprising the roots of the seedlings and growth media) in the single container and to develop the beginnings of vines with true leaves. At this stage, the consumer or end-user can remove the young immature watermelon plants from said container as a single unit with said root ball substantially intact for transplanting either into the ground or into a larger container for further growth, maturation, flowering and ultimately fruit production.

In another embodiment, vegetatively-propagated materials, such as *e.g.* cuttings or plants derived from tissue or cell culture, can be produced using any suitable method in the art.

Any suitable method of vegetative propagation can be used with the present invention. Exemplary methods include cuttings (including rooted and/or unrooted cuttings), vegetative apomixis, layering, division, budding, grafting and/or cell or tissue culture.

In representative embodiments, the vegetatively-propagated material comprises cuttings (rooted and/or unrooted). Cuttings rely on the ability of the plant to grow adventitious roots (*e.g.,* root material that can generate from a location other than the existing or primary root system, for example, from a leaf, stem or vine) under certain conditions. In an exemplary embodiment a vine is rooted at one of its nodes, and the rooted section is then severed.

Many methods of taking vegetative propagation via cuttings are known in the art. In one illustrative method, a cutting is taken with a sharp blade to reduce damage to the tissues. The cutting tool is typically dipped in a disinfectant to prevent disease transmission. Any flowers or flower buds can be removed to direct energy and stored carbohydrates in the cutting to root and short formation. Trimming the leaf (*e.g.,* up to half its length) can be used to improve efficiency. If rooted cuttings are used, a rooting hormone, optionally containing a fungicide, can be used to enhance root formation. Stem and leaf cuttings are typically placed in bright, indirect light. Root cuttings can be kept in the dark until new shoots appear.

In other representative embodiments, the vegetatively-propagated material comprises plantlets grown from cell or tissue culture. Cell or tissue culture relies on the ability of a cell or a tissue to regenerate into a plantlet when cultured on an appropriate medium.

Many methods of regenerating plantlets from cell or tissue are known in the art. In one illustrative example, plantlets can be regenerated from cotyledon and hypocotyl explants of watermelon. The explants are cultured on a Murashige and Skoog's basal nutrient medium supplemented with auxin, cytokinin and auxin-cytokinin combinations. Green healthy nodular and compact callus are obtained in medium containing naphthalene acetic acid and benzylaminopurine. Shoot differentiation and root differentiation from the cotyledon and hypocotyl after callus formation in different media containing benzylaminopurine or naphthalene acetic acid, respectively. Shoot formation requires benzylaminopurine. Root differentiation occurs in a medium containing naphthalene acetic acid or indole acetic acid. There is a greater proliferation of roots on medium supplemented with naphthalene acetic acid. The regenerated shoots develop roots when transferred to medium containing naphthalene acetic acid and complete plantlets can be transferred to soil for further growth.

As used herein, substantially intact" means that the seedlings can be removed from the grow cell or container together with about 25% to about 100% (*e.g.,* at least about 25%, 30%, 35%, 40%, 45%, 50%, 55%, 60%, 65%, 70%, 75%, 80%, 85%, 90%, 95%, 100%, and any amount or range therein) of the growing media remaining with the root ball.

Accordingly, in particular embodiments, the method for producing watermelon fruit according to the present invention further comprises growing the at least one seedling of a seedless watermelon variety and at least one seedling of a seeded pollenizer watermelon variety in a single container for sufficient time to develop into young immature watermelon plants and to allow the roots of said young immature plants in said container to form a root ball. In some embodiments, the method further comprises transplanting the young immature watermelon plants from said single container into the ground or into a larger container, wherein the young immature plants are removed from said container as a single unit with the root ball remaining substantially intact for planting into the ground or into a larger container.

A container useful with this invention can be any container that can hold the designated number of seedlings/plants and sufficient planting media to result in an established group of plants having a common root ball that can be planted in the ground or into a larger container as a single unit. A container can be any useful shape or size such as a round, square or decorative (including, for example, hanging baskets). Thus, for example, a container can be a pot having a diameter of about 3 inches to about 30 inches (*e.g.,* about 3, 4, 5, 6, 7, 8, 9, 10, 11, 12, 13, 14, 15, 16, 17, 18, 19, 20, 21, 22, 23, 24, 25, 26, 27, 28, 29 or 30 inches, and the like), and any size or range therein. In some embodiments, a container can be about 7 cm³ to about 240 cm³ (*e.g.,* about 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240 cm³, and the like) and size or any range therein. In some embodiments, a container can be about a pint to about 30 gallons in size (*e.g.,* 1 pint, 1 quart, or 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 gallons, and the like and any size or range therein). In representative embodiments, a container useful with this invention can be from about 5 cm³ to about 50 cm³ (*e.g.,* about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50 cm³ and the like, and any value or range therein).

Containers can be made of traditional materials (*e.g*., plastic, glass, wood, and the like) or any material appropriate for growing plants and can be non-biodegradable or biodegradable.

A "larger container" can be any container that will allow the seedlings/plants from a single container of this invention to continue to grow and mature to produce edible fruit when transplanted together from the single container. Thus, a "larger container" can be about 1 inch or more or about 2 cm³ or more than the single container that the seedlings/plants were previously planted in.

The present invention is more particularly described in the following examples that are intended as illustrative only since numerous modifications and variations therein will be apparent to those skilled in the art.

### EXAMPLES

These examples describe specific watermelon varieties but as the skilled horticulturist would readily understand these are only exemplary watermelon varieties. Any other appropriate seeded pollenizer watermelon varieties or seedless watermelon varieties known or later developed could be used, including but not limited to those described herein.

### Example 1. Preparing seedlings for containers

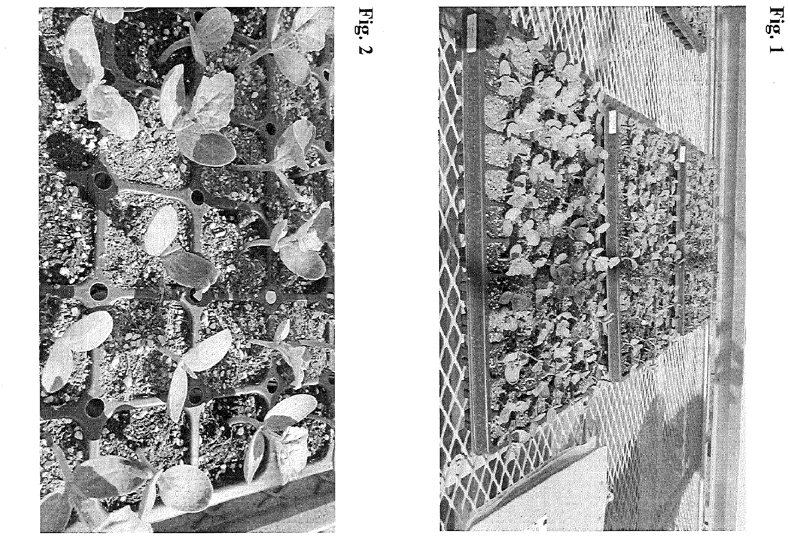

Watermelon seedlings of selected seeded pollenizer variety(ies) and seedless watermelon variety(ies) are germinated and grown in plug trays having cells with a size of about 30 to 50 cm³ containing a soilless soil mix of 50 to 65% high grade peat and 35 to 50% horticultural vermiculite or horticultural perlite. One seed per cell is sown about 2-cm deep. The planting media is generally pre-watered lightly prior to seeding to bring the seed and mix in contact. Trays are then placed under controlled humidity and temperature for 24-48 hours by covering and placing in a germination chamber at 30-35 C. The trays are then arranged on benches in a greenhouse with day temperature 21-27C and night temperature 18-21C where temperature control can be achieved (See, **Fig. 1** and **Fig. 2**).

In each case, the seedlings of the seeded pollenizer watermelon variety(ies) and the seedless watermelon variety(ies) are grown for a sufficient time under the appropriate conditions to produce roots that are sufficiently developed to permit removal from the cells of a plug tray with the growing media volume substantially intact. This will generally require about four to seven weeks from sowing depending on cell size, light and temperature conditions. Alternatively, the seeds of the seeded pollenizer watermelon variety(ies) and the seedless watermelon variety(ies) can be sown directly into the single container and bypass the need to transplant seedlings into the single container from the plug trays.

### Example 2. Planting individual containers

### Three seedling containers

Single containers are planted with various selected combinations of seeded pollenizer watermelon varieties and seedless watermelon varieties as follows.
(1) One seedling of the seeded pollenizer watermelon variety 'Quetzali' along with two seedlings of a seedless watermelon variety 'Sweet Gem' are planted in a single container (e.g., a four inch pot) with growing medium.
(2) One seedling of the seeded pollenizer watermelon variety 'Quetzali' along with two seedlings of a seedless watermelon variety 'Dorin' ('RWT8212'), are planted in a single container with growing medium.
(3) One seedling of the seeded pollenizer watermelon variety 'Quetzali' along with one seedling each of two different seedless watermelon varieties 'Sweet Gem' and 'Dorin' are planted in a single container with growing medium.

### Four seedling containers

(1) Two seedlings of the seeded pollenizer watermelon variety 'Quetzali' along with two seedlings of a single seedless watermelon variety 'Sweet Gem' are planted in a single container with growing medium (*See, e.g.,* **Fig. 3**).
(2) Two seedlings of the seeded pollenizer watermelon variety 'Quetzali' along with two seedlings of a single seedless watermelon variety 'Dorin' are planted in a single container with growing medium (*See, e.g.,* **Fig. 3**).
(3) Two seedlings of the seeded pollenizer watermelon variety 'Quetzali' along with one seedling each of two different seedless watermelon varieties 'Sweet Gem' and 'Dorin' are planted in a single container with growing medium (See, *e.g*., **Fig. 4****;** **Figs. 5A-5F**).

The seedlings in each of the containers are grown for a sufficient time under the appropriate growth conditions to allow the roots of said seedlings in said container to form a root ball (roots of the seedlings intertwined with each other and the growing media) so that the seedlings can be removed from said container as a single unit with the root ball (roots and growing media) substantially intact. This will generally take about two to three weeks from time of planting into the single container (ore about five to about nine weeks if the seed is directly sown into the single container) depending on the size of the single container, as well as light and temperature conditions.

### Example 3. Transplanting the single containers.

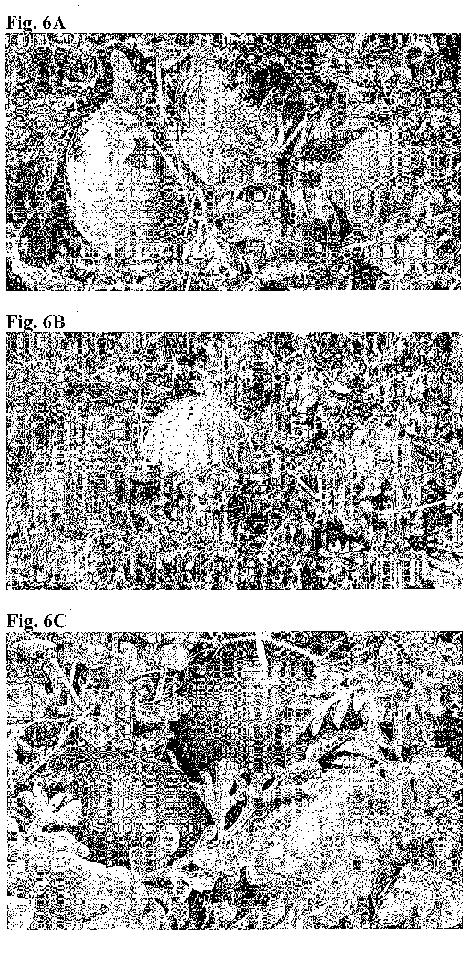

Once the seedlings reach a finished stage, they can then be transplanted into a larger container or into the ground to continue to grow, mature and produce watermelon fruit (*See, e.g.,* **Figs. 6A-6C**). Generally, when transplanted into a larger container the consumer will transplant into a container that is about 24 inches in size or larger.

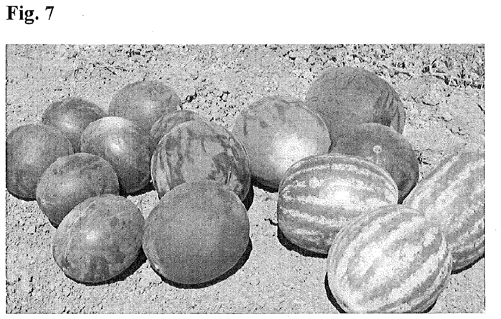

With these unique combinations of seedless and seeded pollenizer watermelon varieties, the consumer or end-user (*e.g.,* gardener, chef, and the like) can produce watermelons throughout an extended season. Thus, in these particular examples, the 'Quetzali' variety and the 'Dorin' variety mature first and thus, produce the first fruits ready for picking and the 'Quetzali' variety will also provide pollen for the seedless variety(ies). The 'Sweet Gem' seedless watermelon variety produces fruit approximately 7 days later than the early season 'Quetzali' and 'Dorin' varieties. As a result, the consumer or end-user will be able to harvest fruit for an extended period from more than one watermelon variety in a single unique offering that a one variety alone cannot offer. Further, the present invention has the additional benefit of providing seeded watermelons as well as seedless watermelons in one offering, which again is something that a single variety cannot offer (*See, e.g.,* **Figs. 6A-6C****;** **Fig. 7**).

Furthermore, as illustrated in these examples, watermelon varieties as described herein can be selected for planting in single containers that produce fruit, which can be visually distinguished allowing the gardener to determine, for instance, which watermelon fruits are seeded and which are seedless. For example, the seeded variety 'Quetzali' produces fruit that has a green and white stripe pattern on the rind, the seedless variety 'Dorin' produces a smaller size fruit with a dark rind and the seedless variety 'Sweet Gem' produces a medium size fruit with a dark rind resulting in a unique combination of different seeded and seedless watermelon fruits, which the home gardener can distinguish (*See, e.g.,* **Figs. 6A-6C****;** **Fig. 7**).

The foregoing is illustrative of the present invention, and is not to be construed as limiting thereof. The invention is defined by the appended claims.

## Claims

1. A method for producing watermelon fruit over an extended season, comprising
planting together in a container at least one seedling of a seedless watermelon variety and at least one seedling of a seeded pollenizer watermelon variety, wherein the at least one seedling of a seeded pollenizer watermelon variety matures in sufficient time to pollenize the at least one seedling of a seedless watermelon variety, thereby producing watermelon fruit over an extended period; and,
transplanting the seedlings into the ground or into a larger container, wherein the seedlings planted in the container form a single root ball and are removed from the single container and planted together into the ground or into a larger container without substantially disrupting said root ball.

2. The method of claim 1, wherein the at least one seedling of a seedless watermelon variety comprises at least two seedlings of at least one seedless watermelon variety.

3. The method of claim 1, wherein the at least one seedling of a seedless watermelon variety consists of a first seedling of a first seedless watermelon variety and a first seedling of a second seedless watermelon variety.

4. The method of claim 3, wherein the at least one seedling of a seeded pollenizer watermelon variety consists of a first and a second seedling of the same seeded pollenizer watermelon variety.

5. The method of claim 2, wherein the at least one seedling of the seeded pollenizer watermelon variety produces male flowers in sufficient time to pollenize female flowers of the at least two seedlings of at least one seedless watermelon variety.

6. The method of claim 4, wherein the first and the second seedling of the same seeded pollenizer watermelon variety produce male flowers in sufficient time to pollenize female flowers of the first seedling of a first seedless watermelon variety and female flowers of the first seedling of a second seedless watermelon variety.

7. The method of claim 1, wherein the seeded pollenizer watermelon variety is an early season variety and the seedless watermelon variety is a mid-season variety or a late season variety.

8. The method of claim 3, wherein the seeded pollenizer watermelon variety is an early season variety, the first seedless watermelon variety is a mid-season variety and the second seedless watermelon variety is a late season variety.

## Patentansprüche

1. Verfahren zur Erzeugung von Wassermelonen über eine verlängerte Saison, umfassend
gemeinsames Pflanzen wenigstens eines Keimlings einer kernlosen Wassermelonensorte und wenigstens eines Keimlings einer kernhaltigen Pollenspender-Wassermelonensorte in einem Behälter, wobei der wenigstens eine Keimling einer kernhaltigen Pollenspender-Wassermelonensorte hinreichend schnell heranreift, so dass der wenigstens eine Keimling einer kernlosen Wassermelonensorte bestäubt wird, wodurch Wassermelonen über eine verlängerte Saison erzeugt werden; und
Umpflanzen der Keimlinge in den Boden oder in einen größeren Behälter, wobei die im Behälter gepflanzten Keimlinge einen einzigen Wurzelballen bilden und gemeinsam aus dem Einzelbehälter genommen und in den Boden oder in einen größeren Behälter gepflanzt werden, ohne den Wurzelballen wesentlich zu stören.

2. Verfahren nach Anspruch 1, wobei der wenigstens eine Keimling einer kernlosen Wassermelonensorte wenigstens zwei Keimlinge wenigstens einer kernlosen Wassermelonensorte umfasst.

3. Verfahren nach Anspruch 1, wobei der wenigstens eine Keimling einer kernlosen Wassermelonensorte aus einem ersten Keimling einer ersten kernlosen Wassermelonensorte und einem ersten Keimling einer zweiten kernlosen Wassermelonensorte besteht.

4. Verfahren nach Anspruch 3, wobei der wenigstens eine Keimling einer kernhaltigen Pollenspender-Wassermelonensorte aus einem ersten Keimling und einem zweiten Keimling der gleichen kernhaltigen Pollenspender-Wassermelonensorte besteht.

5. Verfahren nach Anspruch 2, wobei der wenigstens eine Keimling der kernhaltigen Pollenspender-Wassermelonensorte männliche Blüten hinreichend schnell produziert, so dass weibliche Blüten der wenigstens zwei Keimlinge wenigstens einer kernlosen Wassermelonensorte bestäubt werden.

6. Verfahren nach Anspruch 4, wobei der erste und der zweite Keimling der gleichen kernhaltigen Pollenspender-Wassermelonensorte männliche Blüten hinreichend schnell produziert, so dass weibliche Blüten des ersten Keimlings einer ersten kernlosen Wassermelonensorte und weibliche Blüten des ersten Keimlings einer zweien kernlosen Wassermelonensorte bestäubt werden.

7. Verfahren nach Anspruch 1, wobei es sich bei der kernhaltigen Pollenspender-Wassermelonensorte um eine frühe Sorte und bei der kernlosen Wassermelonensorte um eine mittelfrühe Sorte oder eine späte Sorte handelt.

8. Verfahren nach Anspruch 3, wobei es sich bei der kernhaltigen Pollenspender-Wassermelonensorte um eine frühe Sorte, bei der ersten kernlosen Wassermelonensorte um eine mittelfrühe Sorte und bei der zweiten kernlosen Wassermelonensorte um eine späte Sorte handelt.

## Revendications

1. Méthode de production de pastèques sur une saison étendue, comprenant
la plantation ensemble dans un conteneur d'au moins une plantule d'une variété de pastèque sans pépins et d'au moins une plantule d'une variété de pastèque pollinisatrice avec pépins, où la au moins une plantule d'une variété de pastèque pollinisatrice avec pépins mûrit suffisamment tôt pour polliniser la au moins une plantule d'une variété de pastèque sans pépins, produisant ainsi des pastèques sur une période étendue ; et
le repiquage des plantules dans la terre ou dans un conteneur plus grand, où les plantules plantées dans le conteneur forment une motte racinaire unique et sont retirées du conteneur unique et plantées ensemble dans la terre ou dans un conteneur plus grand sans perturber sensiblement ladite motte racinaire.

2. Méthode selon la revendication 1, dans laquelle la au moins une plantule d'une variété de pastèque sans pépins comprend au moins deux plantules d'au moins une variété de pastèque sans pépins.

3. Méthode selon la revendication 1, dans laquelle la au moins une plantule d'une variété de pastèque sans pépins est constituée d'une première plantule d'une première variété de pastèque sans pépins et d'une première plantule d'une deuxième variété de pastèque sans pépins.

4. Méthode selon la revendication 3, dans laquelle la au moins une plantule d'une variété de pastèque pollinisatrice avec pépins est constituée d'une première et d'une deuxième plantule de la même variété de pastèque pollinisatrice avec pépins.

5. Méthode selon la revendication 2, dans laquelle la au moins une plantule de la variété de pastèque pollinisatrice avec pépins produit des fleurs mâles suffisamment tôt pour polliniser des fleurs femelles des au moins deux plantules d'au moins une variété de pastèque sans pépins.

6. Méthode selon la revendication 4, dans laquelle la première et la deuxième plantule de la même variété de pastèque pollinisatrice avec pépins produisent des fleurs mâles suffisamment tôt pour polliniser des fleurs femelles de la première plantule d'une première variété de pastèque sans pépins et des fleurs femelles de la première plantule d'une deuxième variété de pastèque sans pépins.

7. Méthode selon la revendication 1, dans laquelle la variété de pastèque pollinisatrice avec pépins est une variété de saison précoce et la variété de pastèque sans pépins est une variété de mi-saison ou une variété de saison tardive.

8. Méthode selon la revendication 3, dans laquelle la variété de pastèque pollinisatrice avec pépins est une variété de saison précoce, la première variété de pastèque sans pépins est une variété de mi-saison et la deuxième variété de pastèque sans pépins est une variété de saison tardive.
